# EUROPÄISCHE PATENTSCHRIFT

(11) **EP 2 179 029 B1**
(45) Veröffentlichungstag und Bekanntmachung des Hinweises auf die Patenterteilung: **05.06.2013**
(21) Anmeldenummer: 08784515.2
(22) Anmeldetag: 13.06.2008
(51) Int. Cl.: C12N 5/00, C12M 1/00, C12M 3/00, C12M 1/16

(54) **VERFAHREN UND VORRICHTUNG ZUR BILDUNG VON AGGREGATEN BIOLOGISCHER ZELLEN**
METHOD AND DEVICE FOR FORMING BIOLOGIC CELL AGGREGATES
PROCÉDÉ ET DISPOSITIF POUR LA FORMATION D'AGRÉGATS DE CELLULES BIOLOGIQUES

(30) Priorität: 20.06.2007 DE 102007028423
(43) Veröffentlichungstag der Anmeldung: 28.04.2010
(73) Patentinhaber: Fraunhofer-Gesellschaft zur Förderung der angewandten Forschung e.V., 80686 München (DE)
(72) Erfinder: KRUSE, Charli, 23923 Herrnburg (DE); DANNER, Sandra, 23552 Lübeck (DE)
(74) Vertreter: Hertz, Oliver
(86) Internationale Anmeldenummer: PCT/EP2008/004798
(87) Internationale Veröffentlichungsnummer: WO 2008/155072

(56) Entgegenhaltungen:
- WO-A-97/28252
- WO-A-2005/114178
- DE-A1- 4 132 379
- US-A- 5 650 164
- PARK JAESUNG ET AL: "Microfabrication-based modulation of embryonic stem cell differentiation" LAB ON A CHIP, Bd. 7, Nr. 8, 2007, Seiten 1018-1028, XP002494835 ISSN: 1473-0197(print) 1473-0189(ele
- KARP JEFFREY M ET AL: "Controlling size, shape and homogeneity of embryoid bodies using poly(ethylene glycol) microwells" LAB ON A CHIP, Bd. 7, Nr. 6, Juni 2007 (2007-06), Seiten 786-794, XP002494836 ISSN: 1473-0197(print) 1473-0189(ele
- LIN KONG HUA ET AL: "Long-term maintenance of liver-specific functions in three-dimensional culture of adult rat hepatocytes with a porous gelatin sponge support" BIOTECHNOLOGY AND APPLIED BIOCHEMISTRY, Bd. 21, Nr. 1, 1995, Seiten 19-27, XP009105397 ISSN: 0885-4513
- SODUNKE ET AL: "Micropatterns of Matrigel for three-dimensional epithelial cultures" BIOMATERIALS, ELSEVIER SCIENCE PUBLISHERS BV., BARKING, GB, Bd. 28, Nr. 27, Juli 2007 (2007-07), Seiten 4006-4016, XP022153650 ISSN: 0142-9612
- FUKUDA JUNJI ET AL: "Orderly arrangement of hepatocyte spheroids on a microfabricated chip." TISSUE ENGINEERING 2005 JUL-AUG, Bd. 11, Nr. 7-8, Juli 2005 (2005-07), Seiten 1254-1262, XP002494837 ISSN: 1076-3279
- POLLOK J M ET AL: "FORMATION OF SPHEROIDAL AGGREGATES OF HEPATOCYTES ON BIODEGRADABLE POLYMERS UNDER CONTINUOUS-FLOW BIOREACTOR CONDITIONS" EUROPEAN JOURNAL OF PEDIATRIC SURGERY, HIPPOCRATES VERLAG, STUTTGART, DE, Bd. 8, Nr. 4, 1. August 1998 (1998-08-01), Seiten 195-199, XP001070336 ISSN: 0939-7248
- NAPOLITANO ANTHONY P ET AL: "Dynamics of the self-assembly of complex cellular aggregates on micromolded nonadhesive hydrogels." TISSUE ENGINEERING AUG 2007, Bd. 13, Nr. 8, August 2007 (2007-08), Seiten 2087-2094, XP002494838 ISSN: 1076-3279
- KONNO T ET AL: "Photo-immobilization of a phospholipid polymer for surface modification" BIOMATERIALS, ELSEVIER SCIENCE PUBLISHERS BV., BARKING, GB, Bd. 26, Nr. 12, 1. April 2005 (2005-04-01), Seiten 1381-1388, XP004598662 ISSN: 0142-9612
- FOLCH A ET AL: "Microengineering of cellular interactions." ANNUAL REVIEW OF BIOMEDICAL ENGINEERING 2000, Bd. 2, 2000, Seiten 227-256, XP002494839 ISSN: 1523-9829

## Beschreibung

Die Erfindung betrifft ein Verfahren zur Kultivierung von biologischen Zellen, insbesondere ein Verfahren zur Bildung von biologischen Zellaggregaten, und eine Zellkultivierungsvorrichtung.

Es ist allgemein bekannt, biologische Zellen außerhalb eines Organismus zu kultivieren (in vitro-Kultivierung). Typischerweise bilden die Zellen auf einem Kunststoff- oder Glassubstrat in einem Kultivierungsmedium eine Zellkultur. Auf dem Substrat ist beispielsweise ein Wachstum und/oder eine Differenzierung der Zellen vorgesehen. Bei zahlreichen zellbiologischen Verfahren besteht die Aufgabe, aus der Zellkultur Zellgruppen zu entnehmen, beispielsweise um diese einer weiteren Kultivierung oder einer Untersuchung zu unterziehen. Bei herkömmlichen Standardverfahren der Zellbiologie erfolgt die Entnahme von Zellgruppen aus einer Zellkultur mit mechanischen Mitteln (z. B. mit einem Trennwerkzeug) oder mit chemischen Mitteln (z. B. durch einen enzymatischen Abbau).

Bei der in vitro-Kultivierung auf einem Substrat bilden die Zellen typischerweise eine Zellschicht mit wenigen Zelllagen oder nur einer einzigen Zelllage (Monoschicht). Die Bildung dünner Schichten ergibt ein Problem, wenn für die Untersuchung oder weitere Kultivierung große Zellgruppen erforderlich sind. In diesem Fall müssen die Zellen relativ großflächig vom Substrat getrennt werden. Dies kann zu unerwünschten biochemischen Reaktionen führen, welche die nachfolgenden Verfahrensschritte beeinträchtigen.

Für bestimmte Zelltypen oder Kultivierungsmedien kann in Einzelfällen ein Wachstum über die Monoschicht hinaus erzielt werden. Die Zellen bilden dreidimensionale Zellaggregate. Beispielsweise bilden Stammzellen im adhärenten Zustand Zellaggregate (so genannte organoide Körper, siehe C . Kruse et al. in "Appl. Phys A" Bd. 79, 2004, S. 1617-1624; und C. Kruse et al. in "Ann. Anat." Bd. 188(6) 2006, S. 503-517).

Um dreidimensionale Zellaggregate aus einer Zellkultur zu trennen, können ebenfalls die genannten Standardverfahren der Zellbiologie angewendet werden. Dabei hat sich jedoch in der Praxis das Problem ergeben, dass die Zellaggregate auf dem Substrat nicht gleichförmig gebildet sind. Die Zellaggregate bilden auf den herkömmlich verwendeten Substraten insbesondere in Bezug auf die geometrische Anordnung und Größe der Zellaggregate eine inhomogene Verteilung. Des weiteren befinden sich die typischerweise spontan gebildeten Zellaggregate in verschiedenen Entwicklungsstadien. So kann ein Zellaggregat bestimmte differenzierte Zellen enthalten, während ein jüngeres Zellaggregat diese Zellen nicht enthält. In der Praxis besteht jedoch häufig das Interesse, für nachfolgende Verfahrensschritte eine Vielzahl von Zellaggregaten aus einer Zellkultur zu entnehmen, die möglichst ähnliche Eigenschaften, insbesondere möglichst die gleiche Größe und Form und das gleiche Entwicklungsstadium aufweisen. So werden bei Hochdurchsatz-Testverfahren, bei denen z. B. die biologische Wirkung einer pharmakologischen Substanz untersucht wird, hunderte oder tausende gleichartige Zellproben benötigt.

Eine weitere Beschränkung der herkömmlichen Kultivierungstechniken betrifft die Form der Zellaggregate. Für die Bereitstellung von Gewebemodellen für Untersuchungszwecke oder für die Bildung von Implantaten beim so genannten Tissue Engineering werden Zellaggregate zu größeren Aggregatformationen zusammengesetzt. Dabei besteht ein Interesse, dass die Zellaggregate eine bestimmte Form haben, um die Zusammensetzung der Aggregatformation zu erleichtern. Die Formung von Zellaggregaten ist jedoch mit den herkömmlichen mechanischen Mitteln nur mit großem Aufwand möglich, während eine Formung beim enzymatischen Abbau von Zellen aus einer Zellkultur nahezu ausgeschlossen ist.

Mit den herkömmlichen Standardverfahren der Zellbiologie sind somit lediglich kleine Zellgruppen, die für weitere Verfahrensschritte zu klein sind, oder Zellaggregate, die eine unerwünschte Heterogenität der Eigenschaften aufweisen, aus einer Zellkultur zu gewinnen.

Es ist auch bekannt, biologische Zellen auf porösen Substraten zu kultivieren (z. B. EP 0 759 064 B1, WO 97/00314). Ein poröses Substrat ermöglicht die Beeinflussung der Zellen z. B. mit einer Wirksubstanz von der Substratseite her. Die herkömmlichen porösen Substrate weisen derart geringe Porengrößen auf, dass eine Zelle eine Pore überdeckt (siehe z. B. DE 10 2004 062 216 A1). Die Handhabung der Zellen auf porösen Substraten ist mit den selben Problemen verbunden, die oben zu den Standardverfahren der in vitro-Kultivierung genannt sind.

Park et al. offenbaren in "Lab on a Chip", Bd. 7, Nr. 8, 1018-1028 (2007), die Verwendung von adhäsiven Schablonen zur Herstellung von embryonalen Mausstammzellaggregaten.

Karp et al. beschreiben in "Lab on a Chip", Bd. 7. Nr. 6, 786-794 (2007), die Kultivierung von embryonalen Stammzellen in Mikrovertiefungen, die mit PEG beschichtet sind.

US-A-5 650 164 (1997) offenbart die Kultivierung von Zellen auf perforierten biokompatiblen Membranen.

Lin Kong Hua et al. beschreiben in "Biotechnology and Applied Biochemistry, Bd. 21, Nr. 1, 19-27 (1995), Langzeitkulturen von Hepatozyten auf einem porösen Gelatineschwamm als Substrat.

WO 2005/114178 A (2005) beschreibt ein Verfahren zum Testen von Substanzen unter Verwendung von Zellaggregaten.

Die Aufgabe der Erfindung ist es, ein verbessertes Verfahren zur Kultivierung von biologischen Zellen bereitzustellen, mit dem die Probleme und Beschränkungen herkömmlicher Verfahren überwunden werden. Die Aufgabe der Erfindung ist es auch, eine verbesserte Vorrichtung zur Kultivierung von biologischen Zellen bereitzustellen, mit der Nachteile von herkömmlichen Kultivierungstechniken überwunden werden.

Diese Aufgaben werden durch ein Verfahren bzw. eine Zellkultivierungsvorrichtung mit den Merkmalen der unabhängigen Ansprüche gelöst. Vorteilhafte Ausführungsformen und Anwendungen der Erfindung ergeben sich aus den abhängigen Ansprüchen.

Gemäß einem ersten Gesichtspunkt wird die Aufgabe der Erfindung durch die allgemeine technische Lehre gelöst, ein Verfahren zur Kultivierung von biologischen Zellen bereitzustellen, bei dem auf einem Substrat, dessen Oberfläche Substratöffnungen aufweist, aus den Zellen Zellaggregate gebildet werden, die in den Substratöffnungen angeordnet sind. Die Erfinder haben festgestellt, dass Substrate mit Substratöffnungen so von lebenden Zellen überwachsen werden, dass Gruppen von Zellen die Substratöffnungen überspannen. Die Gruppen von Zellen bilden Zellaggregate. Die Zellaggregate, die eine Zusammensetzung der biologischen Zellen, z. B. in Form organoider Körper umfassen, sind an den Substratöffnungen freitragend gebildet. Jeweils eine der Substratöffnungen wird von einer Gruppe von Zellen überspannt, die ein Zellaggregat bilden.

Vorteilhafterweise werden durch die geometrischen Eigenschaften des Substrats, insbesondere die Größe und Verteilung der Substratöffnungen, Kultivierungsbedingungen gebildet, mit denen die Größe und Verteilung der Zellaggregate beeinflusst, insbesondere bestimmt werden. Im Gegensatz zu der inhomogenen Bildung von Zellaggregaten auf herkömmlichen Substraten mit geschlossenen Oberflächen ergibt die erfindungsgemäße Zellkultivierung eine Verteilung der Zellaggregate entsprechend der Anordnung der Substratöffnungen. Des Weiteren hat sich vorteilhafterweise gezeigt, dass die Zellaggregate in den Substratöffnungen jeweils in einer ähnlichen Größe wachsen können. Bei gleichzeitiger Bereitstellung der Zellen auf dem Substrat können sich die Zellaggregate gleichzeitig entwickeln. Erfindungsgemäß können somit gleichartige Zellaggregate definierter Größe und/oder Entwicklungsstadien in großer Anzahl parallel hergestellt werden, wie sie beispielsweise für Untersuchungsaufgaben, insbesondere bei Hochdurchsatz-Tests erforderlich sind.

Gemäß einem zweiten Gesichtspunkt wird die Aufgabe der Erfindung durch die allgemeine technische Lehre gelöst, eine Zellkultivierungsvorrichtung bereitzustellen, die ein Substrat mit Substratöffnungen und Zellaggregate aufweist, welche die Substratöffnungen überspannen. Vorteilhafterweise bildet die erfindungsgemäße Zellkultivierungsvorrichtung ein Reservoir von Zellaggregaten mit vorbestimmten geometrischen Eigenschaften. Die Zellkultivierungsvorrichtung kann als Probenquelle für zellbiologische Verfahren, wie z. B. die Zellkultivierung, insbesondere die Bildung von Aggregatformationen, oder Zelluntersuchungen verwendet werden.

Mit dem Begriff "Substrat" wird hier allgemein ein Bauteil bezeichnet, das als Träger für biologische Zellen geeignet ist. Das Substrat umfasst einen Substratkörper mit mindestens einer Substratoberfläche, die zur Aufnahme der biologischen Zellen eingerichtet ist und von den Substratöffnungen durchbrochen ist. Teilbereiche des Substratkörpers, die an die Substratöffnungen angrenzen, werden hier auch als Substratelemente bezeichnet. Der Substratkörper hat eine flächige Ausdehnung, er besteht aus einem festen Material, das starr oder nachgiebig sein kann.

Gemäß einer bevorzugten Ausführungsform des erfindungsgemäßen Kultivierungsverfahrens ist eine Trennung der Zellaggregate von dem Substrat, insbesondere durch ein mechanisches Herauslösen der Zellaggregate aus mindestens einer der Substratöffnungen, vorgesehen. Vorteilhafterweise haben die Zellaggregate im Vergleich zur herkömmlichen Kultivierung auf geschlossenen Substratoberflächen weniger Bindungen zwischen Zellen und dem festen Substratmaterial. Daher werden bei der Trennung der Zellaggregate von dem Substrat erheblich weniger Zellen beschädigt und entsprechend weniger unerwünschte biochemische Reaktionen induziert. Die Zellaggregate sind in ihrem biochemischen Zustand durch die Trennung vom Substrat praktisch unbeeinflusst, was wiederum vorteilhaft für nachfolgende Verfahrensschritte, wie z. B. die weitere Kultivierung oder die Untersuchungen an den Zellaggregaten ist.

Für die Trennung der Zellaggregate von dem Substrat weist die erfindungsgemäße Zellkultivierungsvorrichtung vorzugsweise eine Trenneinrichtung mit mindestens einem Trennwerkzeug, vorzugsweise mit einer Vielzahl von Trennwerkzeugen auf, die zumindest an eine Teilmenge der Substratöffnungen des Substrats angepasst sind. Die Trennwerkzeuge haben eine geometrische Anordnung, die gleich der geometrischen Verteilung mindestens eines Teils der Substratöffnungen ist.

Gemäß einer ersten Variante der Erfindung kann die Trennung der Zellaggregate ein Herauslösen aus den Substratöffnungen umfassen. In diesem Fall werden die Zellaggregate vorteilhafterweise vollständig vom Substratmaterial getrennt. Für das Herauslösen der Zellaggregate weist die Trenneinrichtung vorzugsweise Stempelwerkzeuge auf. Jedes Stempelwerkzeug umfasst einen an einem Werkzeugkörper gebildeten Vorsprung, der zum Durchdrücken eines Zellaggregats aus einer der Substratöffnungen eingerichtet ist.

Das Herauslösen der Zellaggregate aus den Substratöffnungen ermöglicht vorteilhafterweise gemäß einer weiteren Ausführungsform der Erfindung, dass die Substratöffnungen mit weiteren Zellen überwachsen werden, so dass sich nach dem Herauslösen einer ersten Generation von Zellaggregaten und dem neuen Bewuchs mit Zellen eine weitere Generation von Zellaggregaten bildet. Diese können wiederum vom Substrat getrennt und weiteren Verfahrensschritten unterzogen werden. Besonders bevorzugt kann ein Kreisprozess realisiert werden, bei dem ein Endlossubstrat aus einem biegsamen Material an der Trenneinrichtung umläuft. Die Zellaggregate werden in den Substratöffnungen des umlaufenden Substrats wiederholt gebildet und jeweils beim Vorbeitritt an der Trenneinrichtung vom Substrat gelöst.

Gemäß einer zweiten Variante der Erfindung kann die Trennung der Zellaggregate gemeinsam mit Substratelementen erfolgen, die an mindestens eine Substratöffnung angrenzen. In diesem Fall kann die Trenneinrichtung ausschließlich auf die Substratelemente einwirken, so dass eine unerwünschte Beeinflussung der Zellaggregate vermindert werden kann. Bei nachfolgenden Verfahrensschritten können die mit den Zellaggregaten vom Substrat entfernten Substratelemente durch die Zellen resorbiert, mechanisch getrennt oder durch eine separat zugesetzte Substanz aufgelöst werden. Für die Trennung der Zellaggregate in Kombination mit den Substratelementen umfassen die Trennwerkzeuge der Trenneinrichtung vorzugsweise Stanzelemente, die eine Anordnung gleich der geometrischen Verteilung der Substratelemente aufweisen, welche die Substratöffnungen umgeben.

Gemäß einer weiteren Variante der Erfindung kann die Trennung der Zellaggregate vom Substrat mit einer Schneideinrichtung erfolgen, die z. B. ein Skalpell aufweist. Diese Ausführungsform wird bevorzugt mit einem kompakten Substrat realisiert, in dessen Oberfläche die Substratöffnungen als Vertiefungen vorgesehen sind.

Gemäß einer besonders bevorzugten Ausführungsform der Erfindung wird eine Vielzahl von Zellaggregaten gleichzeitig von dem Substrat getrennt. Vorteilhafterweise wird damit ermöglicht, dass sich die Zellaggregate im Zeitpunkt der Trennung sämtlich im gleichen geometrischen und/oder biologischen Zustand befinden. In Abhängigkeit von den Anforderungen der Anwendung der Erfindung können somit auch biologisch gleiche Zellaggregate bereitgestellt werden, deren Anzahl 10, z. B. 50 oder 100, für Hochdurchsatz-Tests sogar 500 oder 1000 übersteigt.

Für Kultivierungsaufgaben werden die vom Substrat getrennten Zellaggregate vorzugsweise auf ein Targetsubstrat überführt und dort einer weiteren Kultivierung (insbesondere Wachstum und/oder Differenzierung) unterzogen. Die Gestalt des Targetsubstrats kann frei gewählt werden. Es kann z. B. eine gemeinsame Substratoberfläche für alle Zellaggregate oder eine Vielzahl separater Kammern aufweisen, die jeweils ein Zellaggregat oder eine Gruppe von Zellaggregaten aufnehmen. Besonders bevorzugt für Anwendungen bei der Gewebebildung ist eine Verbindung der Zellaggregate auf dem Targetsubstrat zu Aggregatformationen.

Gemäß einer vorteilhaften Anwendung der Erfindung werden die Zellaggregate in einem ersten Kultivierungsgefäß gebildet, vom Substrat gelöst und mit dem Targetsubstrat in ein zweites Kultivierungsgefäß umgesetzt, wobei unerwünschte mechanische oder enzymatische Einflüsse auf die Zellen vermieden werden.

Ein weiterer Vorteil der Erfindung basiert auf der großen Flexibilität bei der Auswahl und Gestaltung des Substrats. Beispielsweise kann das Substrat einen kompakten Substratkörper aufweisen, in dessen Substratoberfläche die Substratöffnungen als Vertiefungen gebildet sind. Die Zellaggregate werden in der Substratöffnung frei tragend gebildet. In diesem Fall sind die hohe Stabilität des Substrats und der Schutz des Zellaggregats von der Seite des Substratkörpers her von Vorteil. Alternativ kann das Substrat einen Substratkörper aufweisen, in dem die Substratöffnungen von einer Oberfläche zur anderen Oberfläche durchgehend gebildet sind. Der Substratkörper hat die Form einer geraden oder gekrümmten Platte oder Folie mit durchgehenden Löchern. In diesem Fall werden vorteilhafterweise Wechselwirkungen der Zellen mit dem Substratmaterial minimiert und die Versorgung der Zellen in dem Substratöffnungen mit Kultivierungsmedium erleichtert. Das Kultivierungsmedium kann beidseitig den Zellen zugeführt werden. Das Substrat kann insbesondere die Gestalt von einem Netz oder Gitter aufweisen, dessen Maschen die Substratöffnungen bilden. Des Weiteren können die Substratelemente an die Substratöffnungen angrenzend Sollbruchstellen aufweisen. Diese Varianten sind besonders für die gemeinsame Abtrennung von Zellaggregaten und Substratelementen vom Substrat von Vorteil.

Für die Bildung eines Kreisprozesses mit einem umlaufenden Substrat kann es von Vorteil sein, wenn das Substrat eine Dicke aufweist, die geringer als 3 mm ist. Die geringe Dicke kann die Biegsamkeit des Substrats und bei der Bildung mit durchgehenden Substratöffnungen die Versorgung der Zellen mit Kultivierungsmedium verbessern. Das Substrat ist vorzugsweise aus einem biologisch inerten Material, insbesondere einem Kunststoff, z. B. Nylon, Polystyrol, Polypropylen, Nitrocellulose, Polyethylen-Terephtalat, einem Edelmetall, z. B. Gold oder Platin, oder einem resorbierbaren Material, z. B. Fibrin hergestellt.

Die Größe der Substratöffnungen wird vorzugsweise so gewählt, dass die Zellen von den angrenzenden Substratelementen aus den freien Raum der Substratöffnungen überwachsen können und dass ausreichend Platz für die kultivierten Zellaggregate in den Substratöffnungen gegeben ist. Eine typische Dimension der Substratöffnung (z. B. Durchmesser oder Randlänge) ist beispielsweise im Bereich von 5 µm, vorzugsweise größer als 100 µm, z. B. größer als 150 µm, bis 1 mm gewählt. Ein in der Substratöffnung angeordnetes Zellaggregat kann mindestens 5 Zellen, vorzugsweise mindestens 100 Zellen, z. B. mehr als 1000, wie z. B. 100.000 Zellen enthalten.

Vorteilhafterweise können die Substratöffnungen auch verwendet werden, um die Zellaggregate mit einer vorbestimmten Aggregatform zu bilden. Die Querschnittsform der Substratöffnung wird dem Zellaggregat aufgeprägt. Diese Ausführungsform der Erfindung ist insbesondere für Kultivierungsanwendungen, insbesondere im Tissue Engineering von Vorteil.

Erfindungsgemäß werden vorzugsweise Stammzellen (keine humanen embryonalen Stammzellen), insbesondere adulte Stammzellen, besonders bevorzugt glanduläre adulte Stammzellen auf dem Substrat mit den Substratöffnungen kultiviert. Glanduläre adulte Stammzellen werden z. B. von N. W. Guldner et al. in "Int. J. Artif. Organs" Bd. 29(12), 2006, S. 1158-66; S. Danner et al. in "Mol. Hum. Reprod." Bd. 13(1), 2007, S. 11-20; C. Kruse et al. in "Appl. Phys" Bd. 79, 2004, S. 1617-1624; R. M. Seaberg et al. in "Nat. Biotechnol." Bd. 22(9), 2004, S. 1115-1124; und K. L. Seeberger et al. in "Lab. Invest." Bd. 86(2), 2006, S. 141-153 beschrieben. Die Erfinder haben festgestellt, dass Stammzellen aufgrund der folgenden Eigenschaften besonders gut für die erfindungsgemäße Kultivierung geeignet sind. Erstens haben Stammzellen die Fähigkeit, Substrate zu bewachsen und auf diesem dreidimensionale Aggregate (organoide Körper) zu bilden. Die dreidimensionalen Aggregate können ausgehend von adhärenten Stammzellen laufend nachgebildet werden. Dies ist insbesondere für die wiederholte Bildung von Zellaggregaten an Substratöffnungen von Vorteil.

Die Verwendung von Zellaggregaten, die mit dem erfindungsgemäßen Verfahren gebildet wurden, für einen Hochdurchsatz-Test mit biologisch wirksamen Substanzen, z. B. für einen pharmakologischen Hochdurchsatz-Test, oder eine Gewebekultivierung, z. B. für das Tissue Engineering wird offenbart.

Weitere Einzelheiten und Vorteile der Erfindung werden aus der nachfolgenden Beschreibung bevorzugte Ausführungsformen der Erfindung und den beigefügten Zeichnungen ersichtlich. Es zeigen:
- Figur 1:: eine schematische Seitenansicht einer Ausführungsform der erfindungsgemäßen Zellkultivierungsvorrichtung und der erfindungsgemäßen Bildung von Zellaggregaten;
- Figur 2:: eine photographische Darstellung von Zellaggregaten auf einem Substrat mit Substratöffnungen;
- Figur 3:: eine schematische Seitenansicht einer weiteren Ausführungsform der erfindungsgemäßen Zellkultivierungsvorrichtung;
- Figur 4:: eine schematische Illustration einer weiteren Ausführungsform der erfindungsgemäßen Bildung von Zellaggregaten; und
- Figur 5:: eine schematische Draufsicht auf eine weitere Ausführungsform der erfindungsgemäßen Zellkultivierungsvorrichtung.

Ausführungsformen der Erfindung werden im Folgenden unter beispielhaftem Bezug auf die Bildung von Zellaggregaten aus glandulären Stammzellen an Kunststoffnetzen erläutert. Die Umsetzung der Erfindung ist jedoch nicht auf die Verwendung der glandulären Stammzellen, sondern auch mit anderen Stammzellen oder mit anderen Zelltypen, wie z. B. Vorläuferzellen oder differenzierten Zellen realisierbar. Die Zellaggregate können verschiedene Zelltypen, z. B. Stammzellen und erste differenzierten Zellen enthalten. Einzelheiten der Kultivierung von Stammzellen oder anderen Zelltypen werden hier nicht beschrieben, da diese aus den Standardverfahren der Zellbiologie an sich bekannt sind. Des Weiteren können andere Substrate zur Bildung der Zellaggregate verwendet werden, wie dies oben beschrieben ist.

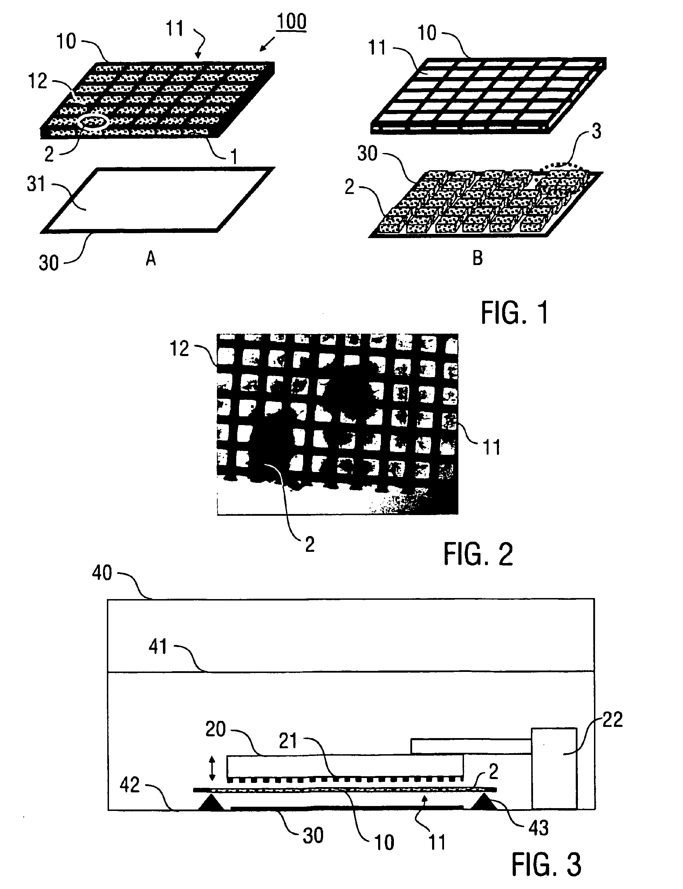

Figur 1A zeigt eine erste Ausführungsform der erfindungsgemäßen Zellkultivierungsvorrichtung 100 mit einem Substrat 10, das eine Vielzahl von Substratöffnungen 11 aufweist, und eine Zellkultur aus biologischen Zellen 1 trägt, die in den Substratöffnungen 11 Zellaggregate 2 bilden. Das Substrat 10 umfasst z. B. ein Nylonnetz, dessen Fäden die Substratelemente 12 bilden, welche die Substratöffnungen 11 umgeben. Die Substratelemente 12 haben einen Durchmesser von z. B. 50 µm. Die Seitenlänge der Substratöffnungen 11 (Maschenweite) beträgt z. B. 0.25 mm. In Figur 1 sind insbesondere die Zellaggregate schematisch gezeigt. In der Realität werden die Zellaggregate typischerweise kugelartig gebildet.

Figur 2 zeigt beispielhaft eine photographische Abbildung mit einer Draufsicht (Teilansicht) auf das erfindungsgemäß verwendete Substrat 10 mit den Substratöffnungen 11, in denen die Zellaggregate 2 gebildet sind. Es ist nicht zwingend erforderlich, dass alle Substratöffnungen 11 Zellaggregate 2 enthalten. Es können, wie in Figur 2 gezeigt, einige Substratöffnungen 11 frei bleiben.

Im unteren Teil von Figur 1A ist ein Targetsubstrat 30 illustriert, das zur Aufnahme von Zellaggregaten 2 aus dem Substrat 10 vorgesehen ist. Das Targetsubstrat 30 weist bei dieser Ausführungsform der Erfindung eine geschlossene, ebene Substratoberfläche 31 auf. Das Substrat 30 besteht z. B. aus Kunststoff oder Glas.

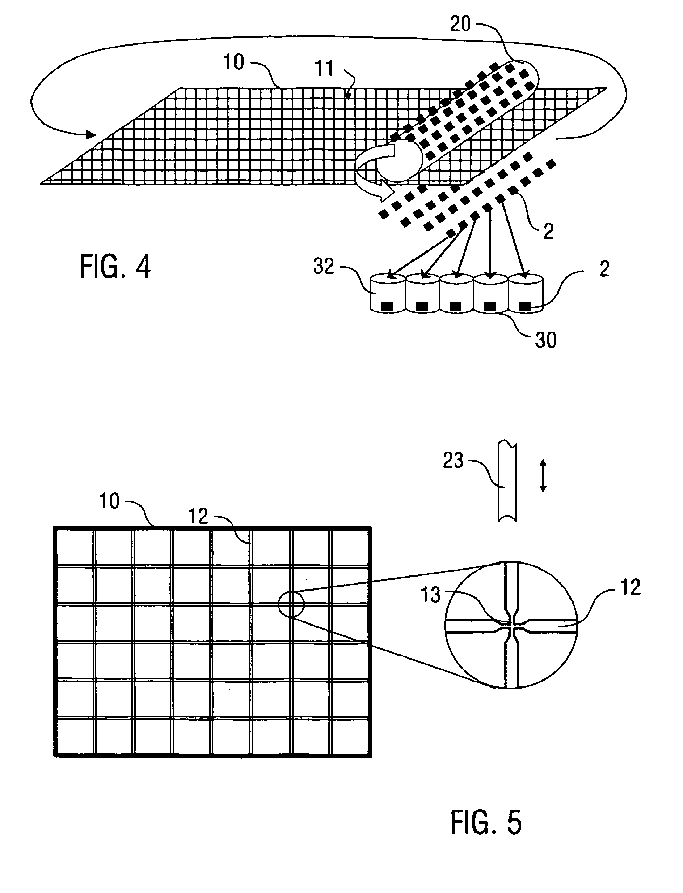

Figur 1B illustriert die Übertragung der Zellaggregate 2 vom Substrat 10 auf das Targetsubstrat 30. Hierzu werden die Zellaggregate 2 einzeln oder gemeinsam mit einer Trenneinrichtung (siehe Figuren 3, 4) aus den Substratöffnungen 11 herausgeschoben, so dass sie auf das Targetsubstrat 30 fallen. Alternativ können die Zellaggregate 2 nach dem Schieben aus den Substratöffnungen auf einer Oberfläche des Substrats 10 angeordnet und von dieser mit einem Schieberelement (nicht dargestellt) aufgenommen und zu dem Targetsubstrat übertragen werden.

Vorzugsweise werden ausschließlich die Zellaggregate aus den Substratöffnungen gelöst, während nach der Trennung Zellen auf dem Substrat verbleiben. Es bleiben z. B. einzelnen Zellen, Zellgruppen oder Monoschichten der Zellen auf dem Substrat, insbesondere den Substratelementen. Diese verbleibenden Zellen (vorzugsweise Stammzellen) können vorteilhafterweise zum erneuten Bewuchs der Substratöffnungen verwendet werden.

Erfindungsgemäß kann des Weiteren vorgesehen sein, dass die vom Substrat 10 abgelösten Zellaggregate auf dem Targetsubstrat 30 miteinander über Zellkontakte aggregieren und Aggregatformationen 3 (schematisch illustriert) bilden.

Figur 3 zeigt eine abgewandelte Variante der erfindungsgemäßen Zellkultivierungsvorrichtung 100 mit dem Substrat 10, das in den Substratöffnungen die Zellaggregate 2 enthält, und einer Trenneinrichtung 20, die eine Vielzahl von Stempelwerkzeugen 21 aufweist. Die Trenneinrichtung 20 ist mit einer schematisch gezeigten Antriebseinrichtung 22 ausgestattet, mit der die Stempelwerkzeuge 21 in die Substratöffnungen des Substrats 10 einführbar sind.

Die Zellkultivierungsvorrichtung 100 ist in einem Kultivierungsgefäß 40 angeordnet, das ein Kultivierungsmedium 41 enthält. Das Substrat 10 ist mit Abstand vom Boden 42 des Kulturgefäßes 42 auf Trägerelementen 43 angeordnet. Auf dem Boden 42 ruht das Targetsubstrat 30 zur Aufnahme der Zellaggregate 2.

Das erfindungsgemäße Verfahren zur Kultivierung biologischer Zellen unter Verwendung der in Figur 3 gezeigten Zellkultivierungsvorrichtung 100 umfasst z. B. die folgenden Schritte. Zunächst erfolgt bei einem Wachstumsschritt ein Wachstum der Zellen auf dem Substrat 10. Die Zellen überwachsen die Substratöffnungen 11. In den Substratöffnungen bilden sich die Zellaggregate 2. In dieser Phase können die Zellaggregate bereits differenzierte Zellen enthalten. Bei einem Trennungsschritt werden die Zellaggregate 2 mit Hilfe der Stempelwerkzeuge 21 vom Substrat 10 gelöst und zum Targetsubstrat 30 überführt.

Eine weitere Variante der erfindungsgemäßen Zellkultivierung ist schematisch in Figur 4 illustriert. Auf dem Substrat 10 werden in den Substratöffnungen 11 die Zellaggregate 2 gebildet. Die Trenneinrichtung 20 umfasst eine Walze, auf deren Oberfläche die Stempelwerkzeuge 21 angeordnet sind. Zur Trennung der Zellaggregate 2 vom Substrat 10 wird dieses an der Walze vorbeigeführt, bei deren Drehung die Stempelwerkzeuge 21 in die Substratöffnungen 11 eingreifen, so dass die Zellaggregate 2 herausgelöst werden. Die Zellaggregate 2 werden zu dem Targetsubstrat 30 überführt, das in diesem Fall mit einer Vielzahl von einzelnen Kammern 32 oder Kompartimenten gezeigt ist.

Mit dem in Figur 4 illustrierten Verfahren kann ein Kreisprozess realisiert werden, in dem das Substrat 10 als umlaufendes Endlosband durch ein Kultivierungsmedium geführt wird. Die Bewegung des Substrats 10 durch das Kultivierungsmedium erfolgt mit einer derartig geringen Geschwindigkeit, dass die gewünschten Zellaggregate 2 bei Erreichen der Trenneinrichtung 20 ausgewachsen sind. Bei der Passage der Trenneinrichtung 20 werden die Zellaggregate 2 zu dem Targetsubstrat 30 übertragen. Anschließend erfolgt ein erneuter Bewuchs der Substratöffnungen 11 mit Zellen, die nach dem Herauslösen der Zellaggregate 2 noch auf den Substratelementen 12 angeordnet sind. Anschließend können die Zellaggregate 2 in den Kammern des in Figur 4 gezeigten Targetsubstrats 30 für eine Kultivierung oder einen Test biologisch oder pharmakologisch wirksamer Substanzen verwendet werden.

Figur 5 zeigt eine weitere Ausführungsform der Erfindung, bei der das Substrat 10 Substratelemente 12 mit Sollbruchstellen 13 umfasst. Die Sollbruchstellen 13 sind, wie in dem vergrößerten Ausschnitt von Figur 5 gezeigt, z. B. an Kreuzungsstellen der Substratelemente 12 in einem Netz oder Gitter gebildet. Zur Trennung der Zellaggregate (in Figur 5 nicht gezeigt) vom Substrat 10 werden die Substratelemente 12 mit einem schematisch gezeigten Stanzwerkzeug 23 an den Sollbruchstellen 13 durchtrennt.

Die in der vorstehenden Beschreibung, den Zeichnungen und den Ansprüche offenbarten Merkmale der Erfindung können sowohl einzeln als auch in Kombination für die Verwirklichung der Erfindung in ihren verschiedenen Ausgestaltungen von Bedeutung sein.

## Patentansprüche

1. Verfahren zur Kultivierung von biologischen Zellen (1), die adulte Stammzellen, Vorläuferzellen und/oder differenzierte Zellen, jedoch keine humanen embryonalen Stammzellen umfassen, mit den Schritten:
- Wachstum der Zellen (1) auf einem Substrat (10), das eine Vielzahl von Substratöffnungen (11) aufweist,
- Bildung von Zellaggregaten (2), die Gruppen der Zellen (1) umfassen, welche jeweils eine der Substratöffnungen (11) überspannen, wobei die Zellaggregate (2) in den Substratöffnungen freitragend angeordnet sind, und
- Trennung der Zellaggregate (2) von dem Substrat (10), wobei die Trennung ein Herauslösen der Zellaggregate (2) aus mindestens einer der Substratöffnungen (11) umfasst.

2. Verfahren nach Anspruch 1, bei dem
- zum Herauslösen der Zellaggregate (2) aus der mindestens einen Substratöffnung (11) ein Stempelwerkzeug (20) verwendet wird.

3. Verfahren nach einem der Anspruche 1 oder 2, bei dem
- die Substratöffnungen (11) nach dem Herauslösen der Zellaggregate (2) mit neuen Zellen besiedelt werden.

4. Verfahren nach Anspruch 3, bei dem
- bei einem Kreisprozess die Zellaggregate (2) wiederholt in den Substratöffnungen (11) eines umlaufenden Substrates (10) gebildet und aus diesen herausgelöst werden.

5. Verfahren nach mindestens einem der vorhergehenden Ansprüche, bei dem
- die Trennung eine Abtrennung von Substratelementen (12), die an mindestens eine Substratöffnung (11) angrenzen, von dem Substrat (10) umfasst.

6. Verfahren nach mindestens einem der vorhergehenden Ansprüche, bei dem
- eine Vielzahl der Zellaggregate (2) gleichzeitig von dem Substrat (10) getrennt werden, und/oder
- eine Bildung einer vorbestimmten Aggregatform der Zellaggregate (2) vorgesehen ist, die durch die Form der Substratöffnungen (11) bestimmt wird.

7. Verfahren nach mindestens einem der vorhergehenden Ansprüche, mit den Schritten
- Überführung der Zellaggregate (2) auf ein Targetsubstrat (30), und
- weitere Kultivierung der Zellaggregate (2) auf dem Targetsubstrat (30).

8. Verfahren nach Anspruch 7, bei dem
- die weitere Kultivierung der Zellaggregate (2) auf dem Targetsubstrat (30) eine Verbindung der Zellaggregate (2) zu Aggregatformationen umfasst.

9. Verfahren nach mindestens einem der vorhergehenden Ansprüche, bei dem das Substrat (10) mindestens eines der Merkmale aufweist:
- das Substrat (10) ist aus einem Kunststoff, inerten Metall oder einem resorbierbaren Material gebildet,
- das Substrat (10) hat eine Dicke, die geringer als 3 mm ist,
- das Substrat (10) besteht aus einem Netz, dessen Maschen die Substratöffnungen (11) bilden,
- das Substrat (10) enthält Substratelemente (12) mit Sollbruchstellen (13),
- die Substratöffnungen (11) sind durch einen Substratkörper durchgehend gebildet,
- die Substratöffnungen (11) sind in der Oberfläche eines Substratkörpers (13) gebildet, und
- die Substratöffnungen (11) haben eine charakteristische Dimension kleiner als 1 mm.

10. Verfahren mindestens einem der vorhergehenden Ansprüche, bei dem
- die adulten Stammzellen glanduläre adulte Stammzellen umfassen.

11. Zellkultivierungsvorrichtung (100), umfassend:
- ein Substrat (10) mit einer Vielzahl von Substratöffnungen (11), die eine vorbestimmte geometrische Verteilung aufweisen,
- Zellaggregate (2), die Gruppen von Zellen (1) umfassen, die adulte Stammzellen, Vorläuferzellen und/oder differenzierte Zellen, jedoch keine humanen embryonalen Stammzellen umfassen, welche jeweils eine der Substratöffnungen (11) freitragend überspannen, und
- eine Trenneinrichtung (20), die eine Vielzahl von Trennwerkzeugen (21, 23) aufweist, deren Anordnung an die geometrische Verteilung der Substratöffnungen (11) angepasst ist.

12. Zellkultivierungsvorrichtung nach Anspruch 11, bei der die Trennwerkzeuge
- Stempel (21) umfassen, deren Anordnung gleich der geometrischen Verteilung der Substratöffnungen (11) ist, oder
- Stanzelemente (23) umfassen, deren Anordnung gleich der geometrischen Verteilung von Substratelementen (12) ist, die an die Substratöffnungen (11) angrenzen.

13. Zellkultivierungsvorrichtung nach Anspruch 12, bei der
- die Stempel (21) eine Stempelform aufweisen, die gleich der Form der Substratöffnungen (11) ist.

14. Zellkultivierungsvorrichtung nach mindestens einem der Ansprüche 11 bis 13, bei der das Substrat (10) mindestens eines der Merkmale aufweist:
- das Substrat (10) ist aus einem Kunststoff, inerten Metall oder einem resorbierbaren Material gebildet,
- das Substrat (10) hat eine Dicke, die geringer als 3 mm ist,
- das Substrat (10) besteht aus einem Netz, dessen Maschen die Substratöffnungen (11) bilden,
- das Substrat (10) enthält Substratelemente (12) mit Sollbruchstellen (13),
- die Substratöffnungen (11) sind durch einen Substratkörper durchgehend gebildet, und
- die Substratöffnungen (11) haben eine charakteristische Dimension kleiner als 1 mm.

## Claims

1. A method for culturing biological cells (1), which comprise adult stem cells, precursor cells and/or differentiated cells but no human embryonic stem cells, comprising the steps:
- growth of the cells (1) on a substrate (10) having a plurality of substrate openings (11), and
- formation of cell aggregates (2) comprising groups of cells (1), each of which spans one of the substrate openings (11), with the cell aggregates (2) being self-supportingly arranged in the substrate openings, and
- separation of the cell aggregates (2) from the substrate (10), with the separation comprising an extraction of the cell aggregates (2) from at least one of the substrate openings (11).

2. The method according to claim 1, wherein
- a stamping tool (20) is used for the extraction of the cell aggregates (2) from the at least one substrate opening (11).

3. The method according to one of claims 1 or 2, wherein
- the substrate openings (11) are populated with new cells after the extraction of the cell aggregates (2).

4. The method according to claim 3, wherein
- in a cyclic process the cell aggregates (2) are repeatedly formed in the substrate openings (11) of a circulating substrate (10) and extracted therefrom.

5. The method according to at least one of the preceding claims, wherein
- the separation comprises a detachment from the substrate (10) of substrate elements (12) adjoining at least one substrate opening (11).

6. The method according to at least one of the preceding claims, wherein
- a plurality of the cell aggregates (2) are simultaneously separated from the substrate (10), and/or
- formation of a predetermined aggregate shape of the cell aggregates (2), which shape is determined by the shape of the substrate openings (11), is provided.

7. The method according to at least one of the preceding claims, comprising the steps:
- transfer of the cell aggregates (2) onto a target substrate (30), and
- further culturing of the cell aggregates (2) on the target substrate (30).

8. The method according to claim 7, wherein
- the further culturing of the cell aggregates (2) on the target substrate (30) comprises joining of the cell aggregates (2) into aggregate formations.

9. The method according to at least one of the preceding claims, wherein the substrate (10) has at least one of the features:
- the substrate (10) is made from a plastic, an inert metal or a resorbable material,
- the substrate (10) has a thickness of less than 3 mm,
- the substrate (10) consists of a net, the meshes of which forming the substrate openings (11),
- the substrate (10) includes substrate elements (12) having predetermined breaking points (13),
- the substrate openings (11) are formed as passing through a substrate body,
- the substrate openings (11) are formed in the surface of a substrate body (13), and
- the substrate openings (11) have a characteristic dimension smaller than 1 mm.

10. The method according to at least one of the preceding claims, wherein
- the adult stem cells comprise glandular adult stem cells.

11. A cell culturing device (100), comprising:
- a substrate (10) with a plurality of substrate openings (11) having a predetermined geometric distribution, and
- cell aggregates (2) comprising groups of cells (1), which comprise adult stem cells, precursor cells and/or differentiated cells but no human embryonic stem cells, each of which spans one of the substrate openings (11) self-supportingly, and
- a separating device (20) with a plurality of separating tools (21, 23), the arrangement of which is adapted to the geometric distribution of the substrate openings (11).

12. The cell culturing device according to claim 11, wherein
- the separating tools comprise stamps (21), the arrangement of which is identical to the geometric distribution of the substrate openings (11), or punching elements (23), the arrangement of which is identical to the geometric distribution of substrate elements (12) surrounding the substrate openings (11).

13. The cell culturing device according to claim 12, wherein
- the stamps (21) have a stamp shape identical to the shape of the substrate openings (11).

14. The cell culturing device according to at least one of claims 11 to 13, wherein the substrate (10) has at least one of the features:
- the substrate (10) is made from a plastic, an inert metal or a resorbable material,
- the substrate (10) has a thickness of less than 3 mm,
- the substrate (10) consists of a net, the meshes of which forming the substrate openings (11),
- the substrate (10) includes substrate elements (12) having predetermined breaking points (13),
- the substrate openings (11) are throughgoingly formed through a substrate body, and
- the substrate openings (11) have a characteristic dimension smaller than 1 mm.

## Revendications

1. Procédé de culture de cellules biologiques (1) qui comprennent des cellules souches adultes, des cellules précurseurs et/ou des cellules différenciées, mais ne comprennent pas de cellules souches embryonnaires humaines, comportant les étapes suivantes :
- croissances des cellules (1) sur un substrat (10) qui présente une multiplicité d'ouvertures de substrat (11),
- formation d'agrégats de cellules (2) qui comprennent des groupes de cellules (1) qui couvrent respectivement l'une des ouvertures de substrat (11), les agrégats de cellules (2) étant disposés dans les ouvertures de substrat de façon autoportante, et
- séparation des agrégats de cellules (2) du substrat (10), la séparation comprenant un détachement des agrégats de cellules (2) d'au moins l'une des ouvertures de substrat (11).

2. Procédé selon la revendication 1, dans lequel
- pour détacher les agrégats de cellules (2) de l'au moins une ouverture de substrat (11), on utilise un outil d'estampage (20).

3. Procédé selon l'une des revendications 1 ou 2, dans lequel
- les ouvertures de substrat (11) sont ensemencées avec de nouvelles cellules après le détachement des agrégats de cellules (2).

4. Procédé selon la revendication 3, dans lequel
- dans un processus cyclique, les agrégats de cellules (2) sont à nouveau formés dans les ouvertures de substrat (11) d'un substrat environnant (10) et détachés de celles-ci.

5. Procédé selon au moins l'une des revendications précédentes, dans lequel
- la séparation comprend un retrait des éléments de substrat (12) qui jouxtent au moins une ouverture de substrat (11) du substrat (10).

6. Procédé selon au moins l'une des revendications précédentes, dans lequel
- une multiplicité d'agrégats de cellules (2) sont séparés en même temps du substrat (10), et/ou
- une formation d'une forme d'agrégat prédéterminée des agrégats de cellules (2) est prévue, qui est déterminée par la forme des ouvertures de substrat (11).

7. Procédé selon au moins l'une des revendications précédentes, comportant les étapes suivantes :
- transfert des agrégats de cellules (2) sur un substrat cible (30), et
- poursuite de la culture des agrégats de cellules (2) sur le substrat cible (30).

8. Procédé selon la revendication 7, dans lequel
- la poursuite de la culture des agrégats de cellules (2) sur le substrat cible (30) comprend une liaison des agrégats de cellules (2) en formations d'agrégat.

9. Procédé selon au moins l'une des revendications précédentes, dans lequel le substrat (10) présente au moins l'une des caractéristiques suivantes :
- le substrat (10) est constitué d'une matière plastique, d'un métal inerte ou d'un matériau résorbable,
- le substrat (10) a une épaisseur qui est inférieure à 3 mm,
- le substrat (10) consiste en un treillis dont les mailles forment les ouvertures de substrat (11),
- le substrat (10) contient des éléments de substrat (12) comportant des positions de rupture (13),
- les ouvertures de substrats (11) sont formées par un corps de substrat continu,
- les ouvertures de substrats (11) sont formées dans la surface d'un corps de substrat (13), et
- les ouvertures de substrat (11) ont une dimension caractéristique inférieure à 1 mm.

10. Procédé selon au moins l'une des revendications précédentes, dans lequel
- les cellules souches adultes comprennent des cellules souches adultes glandulaires.

11. Dispositif de culture cellulaire (100), comprenant :
- un substrat (10) comportant une multiplicité d'ouvertures de substrat (11) qui présentent une répartition géométrique prédéterminée,
- des agrégats de cellules (2) qui comprennent des groupes de cellules (1) qui comprennent des cellules souches adultes, des cellules précurseurs et/ou des cellules différenciées, mais ne comprennent pas de cellules souches embryonnaires humaines, qui couvrent respectivement l'une des ouvertures de substrat (11), de façon autoportante, et
- un système de séparation (20) qui présente une multiplicité d'outils de séparation (21, 23) dont la disposition est adaptée à la répartition géométrique des ouvertures de substrat (11).

12. Dispositif de culture cellulaire selon la revendication 11, dans lequel les outils de séparation
- comprennent un poinçon (21) dont la disposition est identique à la répartition géométrique des ouvertures de substrat (11) ou
- comprennent des éléments d'estampage (23) dont la disposition est identique à la répartition géométrique des éléments de substrat (12) qui jouxtent les ouvertures de substrat (11).

13. Dispositif de culture cellulaire selon la revendication 12, dans lequel
- le poinçon (21) présente une forme de poinçon qui est identique à la forme des ouvertures de substrat (11).

14. Dispositif de culture cellulaire selon au moins l'une des revendications 11 à 13, dans lequel le substrat (10) présente au moins l'une des caractéristiques suivantes :
- le substrat (10) est constitué d'une matière plastique, d'un métal inerte ou d'un matériau résorbable,
- le substrat (10) a une épaisseur qui est inférieure à 3 mm,
- le substrat (10) consiste en un treillis dont les mailles forment les ouvertures de substrat (11),
- le substrat (10) contient des éléments de substrat (12) comportant des positions de rupture (13),
- les ouvertures de substrats (11) sont formées par un corps de substrat continu, et
- les ouvertures de substrat (11) ont une dimension caractéristique inférieure à 1 mm.
